# EUROPEAN PATENT APPLICATION

(11) **EP 3 636 261 A1**
(43) Date of publication of application: **15.04.2020**
(21) Application number: 18809579.8
(22) Date of filing: 01.06.2018
(51) Int. Cl.: A61K 31/397, A61P 25/28

(54) **AGENT FOR PREVENTING OR TREATING TAUOPATHY**

(30) Priority: 02.06.2017 JP 2017109886; 30.06.2017 JP 2017128473
(71) Applicant: FUJIFILM Toyama Chemical Co., Ltd., Chuo-ku Tokyo 1040031 (JP)
(72) Inventor: KOBAYASHI Hiroshi, Tokyo 160-0031 (JP); MATSUMOTO Yoshihiko, Tokyo 160-0031 (JP)
(74) Representative: Hoffmann Eitle
(86) International application number: PCT/JP2018/021225
(87) International publication number: WO 2018/221731

(57) **Abstract**

An object of the present invention is to provide a drug and a method which suppress progress of tauopathy such as Alzheimer's disease. 1-(3-(2-(1-Benzothiophen-5-yl)ethoxy)propyl)azetidin-3-ol or a salt thereof has an effect of reducing the amount of phosphorylated tau protein and an effect of reducing the amount of amyloid β protein in the brain parenchyma, and thus is effective as an agent for preventing or treating tauopathy. Tauopathy can be prevented or treated by administering 1-(3-(2-(1-benzothiophen-5-yl)ethoxy)propyl)azetidin-3-ol or a salt thereof.

## Description

### Technical Field

The present invention relates to an agent for preventing or treating tauopathy, comprising 1-(3-(2-(1-benzothiophen-5-yl)ethoxy)propyl)azetidin-3-ol or a salt thereof as an active ingredient.

### Background Art

Dementia is a neurodegenerative disease with significantly reduced cognitive function caused by, for example, brain atrophy and/or cerebrovascular disorder. Dementia is classified into some types by its cause, and 60% to 80% of the patients with dementia suffers from Alzheimer's disease (AD) (Non Patent Literature 1). The pathogenesis of AD is complicated, and the cause is considered to be the formation of senile plaques due to coagulation of amyloid-β protein (Aβ) or neurofibrillary changes caused by coagulation of phosphorylated Tau protein (p-Tau) (Non Patent Literature 2). The number of patients with AD in Japan is estimated to be about more than 1,160,000. The incidence is higher in advanced age, and thus with the aging of society, the number of patients is expected to increase rapidly, causing a greater burden on patients' family and a sharp rise in medical and nursing care expenses in the future (Non Patent Literatures 3, 4). Thus, treatment of AD is important for not only preventing patients' quality of life from decreasing and reducing burden on their family thereafter, but also reducing medical expenses in the future aging society.

Symptoms of dementia include core symptoms of cognitive impairment and peripheral symptoms such as problem behaviors seen when patients with cognitive impairment interact with people around them (Non Patent Literature 5). At present four agents are used as an agent for treating AD in Japan: donepezil hydrochloride, galantamine hydrobromide, and rivastigmine, which are acetylcholinesterase inhibitors, and memantine hydrochloride which is a N-methyl-D-aspartate receptor antagonist. These are all capable of reducing core symptoms or peripheral symptoms. However, these drugs are symptomatic drugs which improve core symptoms or peripheral symptoms for a certain period of time, and do not suppress neurodegeneration in AD. Although these drugs are temporally effective in improving cognitive function at the beginning of use, the cognitive function usually becomes worse than cognitive function before the treatment, after about 48 weeks or more (Non Patent Literature 6).

The amount of Aβ, which is considered to cause the development of AD, is known to be controlled by its production by cleavage of precursor protein and its removal by glial cells in the brain. Aβ is known to accumulate in the brain with age as a soluble oligomer or insoluble aggregate. Soluble Aβ in the brain is incorporated into astrocytes and microglia. On the other hand, Aβ which has become insoluble and been aggregated is phagocytosed by microglia expressing complement receptor and IgG receptor, and excreted into cerebrospinal fluid (CSF), lymph or blood (Non-patent Literature 7). The amount of Aβ in CSF is decreased with the progress of AD (Non-patent Literature 8). This is thought to suggest an increased amount of aggregated Aβ in the brain. A literature on diagnostic criteria of AD describes a reduced amount of Aβ in CSF and an increased accumulation of amyloid tracer in PET imaging as a biomarker of deposition of Aβ in the brain (Non-patent Literature 9).

An increased amount of insoluble Aβ in the brain is known to induce abnormal phosphorylation of Tau, leading to neurodegeneration. It is known that p-Tau is also detected in CSF and the amount of p-Tau in CSF is correlated well with conditions of AD.

Neurodegeneration due to an excessive increase in the amount of p-Tau has been observed in not only AD, but also mild cognitive impairment (MCI), frontotemporal dementia, Pick's disease, progressive supranuclear palsy and corticobasal degeneration, and these diseases are collectively called tauopathy.

1-(3-(2-(1-benzothiophen-5-yl)ethoxy)propyl)azetidin-3-ol (hereinafter referred to as "Compound A") or a salt thereof is known to have neuroprotective, nerve regeneration-promoting and neurite outgrowth actions, and be useful as a therapeutic agent for central and peripheral neurological diseases (Patent Literature 1). Furthermore, a publication discloses that usually the drug may be administered to an adult in a dose or divided doses of 0.01 to 500 mg per day (Patent Literature 2).

### Prior Art Literatures

### Patent Literature

Patent Literature 1: International Publication No. WO 2003/035647
Patent Literature 2: International Publication No. WO 2003/105830

### Non-Patent Literature

Non Patent Literature 1: 2012 Alzheimer's Disease Facts and Figures. (http://www.alz.org/downloads/facts_figures_2012.pdf)
Non Patent Literature 2: YAKUGAKU ZASSHI, 2010, Vol. 130, No. 4, pp. 521-526
Non Patent Literature 3: Japanese Journal of Clinical Medicine, 2008, Vol. 66 (Extra ed. 1), pp. 23-27
Non Patent Literature 4: Press Release by Seed Planning (December 28, 2010) (http://www.seedplanning.cojp/press/2010/2010122801.html)
Non Patent Literature 5: Japanese Journal of Clinical Psychopharmacology, 2011, Vol. 14, No. 7, pp. 1123-1129
Non Patent Literature 6: Japanese Journal of Clinical Psychopharmacology, 2012, Vol. 15, No. 3, pp. 311-321
Non-patent Literature 7: Proceedings of the Annual Meeting of the Japanese Research Group on Senile Dementia, 2010, Vol. 15, pp. 79-81
Non-patent Literature 8: Archives of Neurology, 2011, Vol. 68, No. 10, pp. 1257-1266
Non-patent Literature 9: Japanese Journal of Geriatrics, 2013, Vol. 50, No. 1, pp. 1-8

### Summary of Invention

### Problem to be Solved by the Invention

Drugs which suppress progress of AD by inhibiting neurodegeneration need to be developed early. An object of the present invention is to provide a drug and a method which suppress progress of tauopathy such as AD.

### Means for Solving Problem

In such circumstances, the present inventors have found that Compound A or a salt thereof has an effect of reducing the amount of p-Tau and an effect of reducing the amount of Aβ in the brain parenchyma, and thus is effective in prevention or treatment of tauopathy, and the present invention has been completed.

The present invention provides the following.
(1) An agent for preventing or treating tauopathy, comprising Compound A or a salt thereof as an active ingredient.
(2) The agent for preventing or treating tauopathy according to (1), wherein the agent has an effect of reducing the amount of p-Tau.
(3) The agent for preventing or treating tauopathy according to (2), wherein the amount of p-Tau is an amount of p-Tau in CSF.
(4) The agent for preventing or treating tauopathy according to any one of (1) to (3), wherein the agent has an effect of reducing the amount of Aβ in the brain.
(5) The agent for preventing or treating tauopathy according to any one of (1) to (4), wherein the agent has an effect of increasing the amount of Aβ in CSF.
(6) The agent for preventing or treating tauopathy according to any one of (1) to (5), wherein the agent is orally administered in a dose of 100 mg to 400 mg in terms of Compound A once a day.
(7) The agent for preventing or treating tauopathy according to any one of (1) to (5), wherein the agent is orally administered in a dose of 160 mg or 320 mg in terms of Compound A once a day.
(8) The agent for preventing or treating tauopathy according to any one of (1) to (7), wherein the tauopathy is AD, Probable AD, Possible AD, Preclinical AD, Prodromal AD, MCI due to AD or MCI.
(9) The agent for preventing or treating tauopathy according to any one of (1) to (7), wherein the tauopathy is AD, MCI due to AD or MCI.
(10) The agent for preventing or treating tauopathy according to any one of (1) to (7), wherein the tauopathy is AD.
(11) The agent for preventing or treating tauopathy according to any one of (1) to (7), wherein the tauopathy is a disease excluding AD.
(12) The agent for preventing or treating tauopathy according to (11), wherein the disease excluding AD is MCI due to AD, MCI, frontotemporal dementia, Pick's disease, progressive supranuclear palsy, corticobasal degeneration or Down syndrome.
(13) The agent for preventing or treating tauopathy according to (11), wherein the disease excluding AD is MCI due to AD or MCI.

The present invention also provides the following.
(a) A pharmaceutical composition for preventing or treating tauopathy, comprising Compound A or a salt thereof as an active ingredient.
(b) Compound A or a salt thereof for use in prevention or treatment of tauopathy.
(c) A method of preventing or treating tauopathy, comprising administering Compound A or a salt thereof to a patient.
(d) Use of Compound A or a salt thereof for producing an agent for preventing or treating tauopathy.
(e) An agent for reducing the amount of p-Tau, comprising Compound A or a salt thereof as an active ingredient.
(f) An agent for reducing the amount of Aβ in the brain, comprising Compound A or a salt thereof as an active ingredient.
(g) An agent for increasing the amount of Aβ in CSF, comprising Compound A or a salt thereof as an active ingredient.
(h) Compound A or a salt thereof for use in a therapeutic measure for reducing the amount of p-Tau.
(i) Compound A or a salt thereof for use in a therapeutic measure for reducing the amount of Aβ in the brain.
(j) Compound A or a salt thereof for use in a therapeutic measure for increasing the amount of Aβ in CSF.
(k) A method of reducing the amount of p-Tau, comprising administering Compound A or a salt thereof to a patient.
(l) A method of reducing the amount of Aβ in the brain, comprising administering Compound A or a salt thereof to a patient.
(m) A method of increasing the amount of Aβ in CSF, comprising administering Compound A or a salt thereof to a patient.
(n) Use of Compound A or a salt thereof for producing an agent for reducing the amount of p-Tau.
(o) Use of Compound A or a salt thereof for producing an agent for reducing the amount of Aβ in the brain.
(p) Use of Compound A or a salt thereof for producing an agent for increasing the amount of Aβ in CSF.

### Advantageous Effects of Invention

The amount of p-Tau can be reduced and the amount of Aβ in the brain parenchyma can be reduced by administering Compound A or a salt thereof, and thus tauopathies such as AD can be prevented or treated.

### Brief Description of Drawings

[Figure 1] Figure 1 is a graph showing change in the concentration of Aβ (Aβ-38, Aβ-40 and Aβ-42) in cerebrospinal fluid at week 52 from the baseline. "n.s." means that there was no statistically significant difference.
[Figure 2] Figure 2 is a graph showing change in the concentration of Tau (p-Tau and total-Tau) in the cerebrospinal fluid at week 52 from the baseline. "n.s." means that there was no statistically significant difference.
[Figure 3] Figure 3 is a graph showing the ratio of p-Tau to total-Tau (p-Tau/total-Tau) for the change in the concentration of Tau in cerebrospinal fluid at week 52 from the baseline. "n.s." means that there was no statistically significant difference.
[Figure 4] Figure 4 is a graph showing change in the concentration of Tau (remeasured total-Tau) in cerebrospinal fluid at week 52 from the baseline. "n.s." means that there was no statistically significant difference.
[Figure 5] Figure 5 is a graph showing the ratio of p-Tau to remeasured total-Tau (p-Tau/total-Tau) for the change in the concentration of Tau in cerebrospinal fluid at week 52 from the baseline. "n.s." means that there was no statistically significant difference.

### Embodiments for Carrying out the Invention

Hereinafter the present invention will be described in detail.

In the present description, the respective terms have the following meaning unless otherwise specified.

In the present description, the numerical range shown with "to" represents a range inclusive of the value before and after "to" as the minimum and maximum value, respectively.

Compound A means 1-(3-(2-(1-benzothiophen-5-yl)ethoxy)propyl)azetidin-3-ol.

Examples of salts of Compound A include known salts of a basic group such as amino group or an acidic group such as hydroxyl group or carboxyl group.

Examples of salts of a basic group include salts with a mineral acid such as hydrochloric acid, hydrogen bromide, nitric acid and sulfuric acid; salts with an organic carboxylic acid such as formic acid, acetic acid, citric acid, oxalic acid, fumaric acid, maleic acid, succinic acid, malic acid, tartaric acid, aspartic acid, trichloroacetic acid and trifluoroacetic acid; and salts with a sulfonic acid such as methanesulfonic acid, benzenesulfonic acid, p-toluenesulfonic acid, mesitylenesulfonic acid and naphthalenesulfonic acid.

Examples of salts of an acidic group include salts with an alkali metal such as sodium and potassium; salts with an alkaline earth metal such as calcium and magnesium; ammonium salts; and salts with a nitrogen-containing organic base such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-efenamin and N,N'-dibenzylethylenediamine.

Of the above salts, pharmacologically acceptable salts are preferred, and salts with maleic acid are more preferred.

In the case where Compound A or a salt thereof has isomers (e.g., optical isomers, geometric isomers and tautomers), the present invention includes all these isomers and also includes hydrates, solvates and any crystal forms thereof.

Tauopathy is a disease in which neurodegeneration is caused by an excessive increase in the amount of p-Tau. Examples thereof include AD, Probable AD, Possible AD, Preclinical AD, Prodromal AD, MCI due to AD, MCI, frontotemporal dementia, Pick's disease, progressive supranuclear palsy, corticobasal degeneration and Down syndrome.

In an embodiment of the present invention, examples of diseases excluding AD include MCI due to AD, MCI, frontotemporal dementia, Pick's disease, progressive supranuclear palsy, corticobasal degeneration and Down syndrome, preferably MCI due to AD and MCI.

In an embodiment of the present invention, examples preferably include AD, Probable AD, Possible AD, Preclinical AD, Prodromal AD, MCI due to AD and MCI, more preferably AD, MCI due to AD and MCI, and further preferably AD.

Diagnosis of Probable AD, Possible AD, Preclinical AD, Prodromal AD and MCI due to AD is described in Alzheimer's Dement., May 2011, Vol. 7, No. 3, pp. 263 - 292.

Pathogenesis and progress of tauopathies is known to be related to cerebrospinal fluid (CSF) biomarkers. For example, in Lancet Neurol. February 2013, Vol. 12, No. 2, pp. 207-216, abnormality of CSF biomarkers has shown to reach the maximum at the onset of AD, while the abnormality of CSF biomarkers is relatively small and is increasing at the stage of MCI due to AD.

If the abnormality of CSF biomarkers can be reduced, improved, inhibited or delayed at the stage where the abnormality is relatively small, a relatively higher effect of prevention and treatment is expected. Thus, it is also preferable that the present invention be used for tauopathy in which abnormality of CSF biomarkers is relatively small.

Prevention means to prevent the onset of a specific disease or at least one symptom caused by the disease.

Treatment means to reduce or improve at least one symptom caused by a specific disease with which a subject is affected, and delay the progress of the disease.

In an embodiment of the present invention, prevention means to inhibit or delay the onset or progress of increase in the amount of insoluble Aβ or the amount of p-Tau in the brain in a patient with, for example, tauopathy. Treatment means to inhibit or delay the progress of increase in the amount of insoluble Aβ or the amount of p-Tau in the brain or to reduce the amount of insoluble Aβ or the amount of p-Tau in the brain.

Mild to moderate Alzheimer's disease may be clinically diagnosed as "probable AD" according to the diagnosis criteria provided by the National Institute of Neurological and Communicative Disorders and Stroke/the Alzheimer's Disease and Related Disorders Associations (NINCDS-ADRDA).

A usual doctor may reasonably make clinical diagnosis of "mild to moderate Alzheimer's disease" using standard criteria. For example, according to the score of the standardized Mini-Mental State Examination (MMSE, scores of 0 to 30), clinical diagnosis of mild to moderate, moderate, or moderate to severe AD is provided. The MMSE (Folstein, Folstein and McHugh, 1975) is a simple test of cognitive function including an interview with patients. Orientation, memory, calculation and attention, language skills and other functions are assessed. The total score is 30. The lower the score, the higher the level of impairment of cognitive function.

In Test Examples of the present invention, patients with an MMSE score of 12 to 22 at the start of the test (screening) were determined as mild to moderate AD. Note that the MMSE is not the only way to clinically determine the grade of AD, though convenient.

The relationship between cerebrospinal fluid (CSF) biomarkers and conditions of AD is widely studied. Amyloid β protein (Aβ-38, Aβ-40 and Aβ-42) in CSF may reflect the level of deposition of amyloid in the brain. Furthermore, tau protein (Tau) and phosphorylated tau protein (P-Tau) in CSF may be indicative of the level and progress of neurodegeneration. In a clinically effective treatment for reducing the level of Tau in CSF, a suppressed progress of neurodegeneration may be observed. Change in Aβ may indicate the effect of a drug for metabolism, deposition or elimination of Aβ.

Compound A or a salt thereof used in the present invention may be prepared by a method known per se or by combining such methods, or by the method disclosed in Patent Literature 1.

Compound A or a salt thereof used in the present invention may be blended with various pharmaceutical additives such as an excipient, a binding agent, a disintegrating agent, a disintegration inhibitor, a consolidation/adhesion-preventing agent, a lubricant, an absorption/adsorption carrier, a solvent, a bulking agent, an isotonic agent, a solubilizer, an emulsifier, a suspending agent, a thickener, a coating agent, an absorption enhancer, a gelling/procoagulant agent, a light stabilizer, a preservative, a desiccant, an emulsification/suspension/dispersion stabilizer, a color protecting agent, a deoxidant/antioxidant, a flavoring agent, a coloring agent, a foaming agent, an antifoaming agent, a soothing agent, an antistatic agent, a buffer, and/or a pH adjuster to give a pharmaceutical preparation such as an oral preparation (e.g., tablets, capsules, powders, granules, fine granules, pills, suspensions, emulsions, liquids, and syrups), injections, eye drops, nasal sprays and transdermal agents. Tablets are preferred as an oral dosage form for patients with AD.

The above agents are formulated by a usual method.

The method of administration of Compound A, which is not particularly limited, is accordingly determined based on the form of the preparation, the age, sex and other conditions of the patient and the level of symptoms of the patient.

The dose of Compound A is accordingly selected based on the administration, the age, sex, type of disease and other conditions of the patient.

The agent may be administered to an adult in a dose or divided doses of usually 40 to 500 mg in terms of Compound A per day. The agent is administered in a dose or divided doses of preferably 100 to 400 mg in terms of Compound A per day, and administered in a dose of further preferably 160 mg or 320 mg in terms of Compound A per day.

In the administration of Compound A or a salt thereof in the present invention, prevention or treatment by administration of acetylcholinesterase inhibitors (AChEIs) may also be included. Examples of AchEIs include donepezil hydrochloride, galantamine hydrochloride, rivastigmine tartrate and tacrine hydrochloride.

In the present invention, the subject may have undergone prevention or treatment by administration of AChEI for at least 6 months before administration of Compound A or a salt thereof.

Next, the present invention will be described with reference to Test Examples and Preparation Examples, but the present invention is not limited thereto.

Maleate of Compound A was used as the test compound.

### Test Example 1 Multicenter randomized double-blind phase II placebo-controlled trial for assessing effectiveness and safety of Compound A in mild to moderate AD patients

Subject (selection criteria): Patients were screened in a period from 42 days before treatment assignment to the assignment based on the following selection criteria.
- Patients who were probable AD and are 55 years old or older and 85 years old or younger at the time of obtaining consent of screening.
- Patients with an MMSE score of 12 to 22 at the time of screening
- Patients with a Modified Hachinski Ischemia Scale score of 4 or less
- Patients who have been treated with a donepezil hydrochloride or rivastigmine transdermal system for at least 4 months before the baseline and with a stable dose thereof for 3 months before the baseline.
- In the case of patients who have received memantine in addition to being treated with a donepezil hydrochloride or rivastigmine transdermal system, patients who have been treated with memantine for at least 4 months before the baseline and with a stable dose thereof for 3 months before the baseline.
- Patients whose brain MRI or CT results match AD at the time of screening

Organization of groups: Patients matched (484 patients) were randomly divided into the following 3 groups and the trial was started.
(1) High dose group: 224 mg of a test compound (160 mg in terms of Compound A) was orally administered once a day for 4 weeks and then 448 mg of a test compound (320 mg in terms of Compound A) was orally administered once a day for 48 weeks (158 patients)
(2) Low dose group: 224 mg of a test compound (160 mg in terms of Compound A) was orally administered once a day for 52 weeks (166 patients)
(3) Placebo group: placebo was orally administered once a day for 52 weeks (158 patients) Method of assessment:

### Cerebrospinal fluid biomarker

Cerebrospinal fluid was collected by lumber puncture from subjects at baseline (within 2 weeks before the first day of administration of investigational drug) and after 52 weeks (within 2 weeks before week 52), and divided into 9-ml aliquots in polyethylene tubes and stored at -80°C. The total tau protein concentration (total-Tau) in the cerebrospinal fluid was measured by an ECL method. The phosphorylated tau protein concentration (p-Tau) was measured by ELISA method. The Aβ-42 value in the cerebrospinal fluid was measured by sandwich ELIZA which has been designed for measurement of Aβ including 1 amino acid and 42 amino acids.

The total-Tau concentration was measured twice using the same cerebrospinal fluid sample.

### Statistical analysis:

Change in cerebrospinal fluid (CSF) biomarkers at week 52 from the baseline was compared by analysis of covariance between a high dose group and a placebo group, and between a low dose group and the placebo group. For models, the baseline of cerebrospinal fluid (CSF) biomarkers was included as a covariate and the significance level was 5%.

### Results: shown below

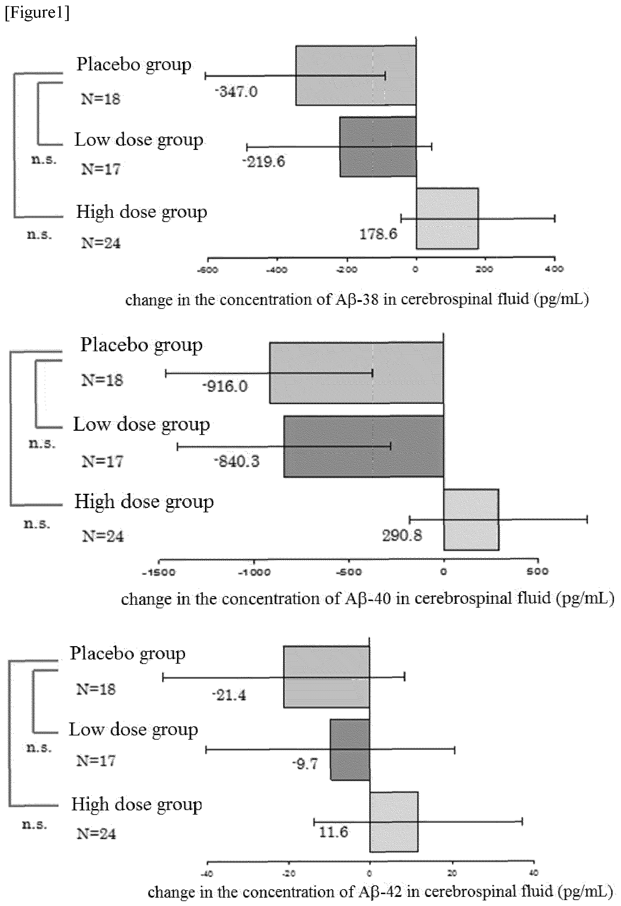

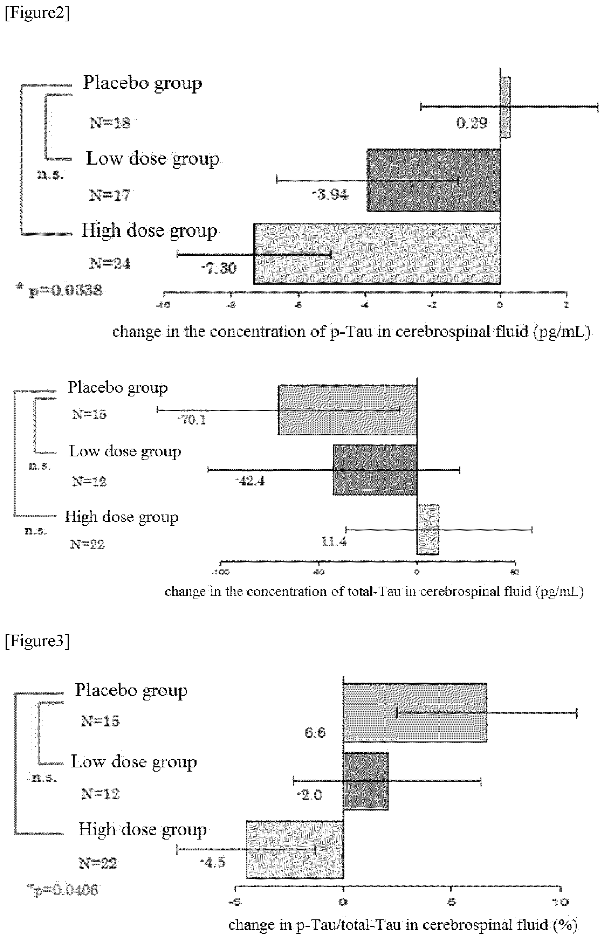

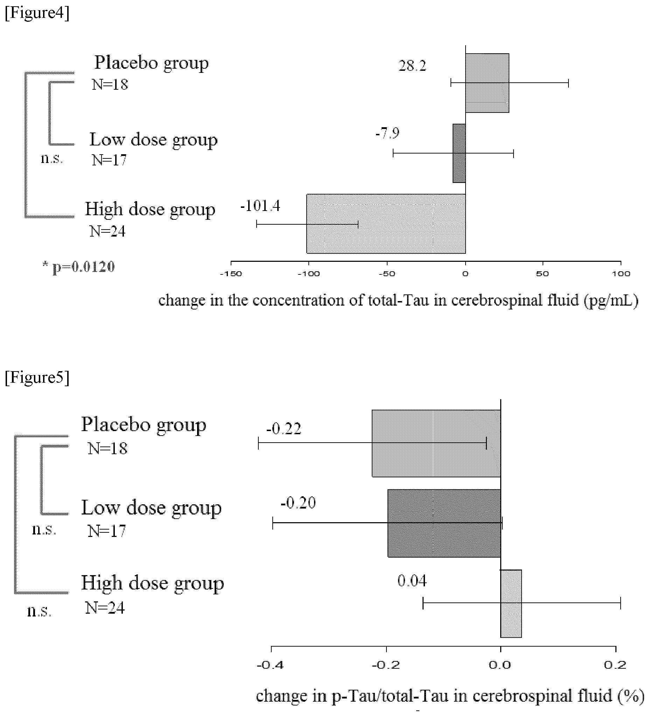

Change in the concentration of cerebrospinal fluid (CSF) biomarkers (Aβ-38, Aβ-40, Aβ-42, p-Tau and total-Tau) at week 52 from the baseline is shown in Table 1, Table 2, Table 3, Figure 1, Figure 2, Figure 3, Figure 4 and Figure 5.

**[Table 1]**

| Group | Number of cases/statistics | Biomarker | | |
|---|---|---|---|---|
| | | Aβ-38(pg/mL) | Aβ-40(pg/mL) | Aβ-42(pg/mL) |
| High dose group | Number of cases | 24 | 24 | 24 |
| | Least square means (standard error) | 178.64(221.978) | 290.84(466.956) | 11.55(25.578) |
| | Difference from placebo group (95% Confidence interval) | 525.66 (-157.19, 1208.50) | 1206.87 (-236.41, 2650.16) | 32.90 (-45.62, 111.41) |
| | p-value | 0.1286 | 0.0995 | 0.4047 |
| Low dose group | Number of cases | 17 | 17 | 17 |
| | Least square means (standard error) | -219.58(266.774) | -840.32(559.467) | -9.70(30.362) |
| | Difference from placebo group (95% Confidence interval) | 127.43 (-625.01, 879.87) | 75.71 (-1507.58, 1659.00) | 11.65 (-73.37, 96.67) |
| | p-value | 0.7356 | 0.9240 | 0.7847 |
| Placebo group | Number of cases | 18 | 18 | 18 |
| | Least square means (standard error) | -347.01(258.755) | -916.03(546.085) | -21.35(29.584) |

For the change in the concentration of Aβ in the cerebrospinal fluid at week 52 from the baseline, the concentration of Aβ tended to be increased in a dose dependent manner in the Compound A group compared to the placebo group.

**[Table 2]**

| Group | Number of cases/statistics | Biomarker | | |
|---|---|---|---|---|
| | | p-Tau (pg/mL) | total- Tau(pg/mL) | p-Tau/total-Tau(%) |
| High dose group | Number of cases | 24 | 20 | 20 |
| | Least square means (Standard error) | -7.30(2.281) | 11.38(47.304) | -4.47(3.21) |
| | Difference from placebo group (95% Confidence interval) | -7.59 (-14.57, -0.60) | 81.94 (-76.34, 240.21) | -11.11 (-21.72, -0.50) |
| | p-value | 0.0338 | 0.3015 | 0.0406 |
| Low dose group | Number of cases | 17 | 11 | 11 |
| | Least square means (Standard error) | -3.94(2.715) | -42.36(63.947) | 2.04(4.33) |
| | Difference from placebo group (95% Confidence interval) | -4.23 (-11.83, 3.37) | 28.19 (-153.16, 209.54) | -4.60 (-16.72, 7.53) |
| | p-value | 0.2692 | 0.7549 | 0.4478 |
| Placebo group | Number of cases | 18 | 12 | 12 |
| | Least square means (Standard error) | 0.29(2.637) | -70.55(61.837) | 6.63(4.15) |

**[Table 3]**

| Total-Tau concentration remeasured. | | | | |
|---|---|---|---|---|
| Group | Number of cases/ statistics | Biomarker | | |
| | | p-Tau(pg/mL) | total-Tau(pg/mL) | p-Tau/total-Tau(%) |
| High dose group | Number of cases | 24 | 24 | 24 |
| | Least square means (Standard error) | -7.30(2.281) | -101.35 (32.552) | 0.04(0.17) |
| | Difference from placebo group (95% Confidence interval) | -7.59 (-14.57, -0.60) | -129.57 (-229.53, -29.60) | 0.26 (-0.28, 0.80) |
| | p-value | 0.0338 | 0.0120 | 0.3368 |
| Low dose group | Number of cases | 17 | 17 | 17 |
| | Least square means (Standard error) | -3.94(2.715) | -7.88 (38.632) | -0.20(0.20) |
| | Difference from placebo group (95% Confidence interval) | -4.23 (-11.83, 3.37) | -36.10 (-144.30, 72.11) | 0.03 (-0.54, 0.59) |
| | p-value | 0.2692 | 0.5066 | 0.9224 |
| Placebo group | Number of cases | 18 | 18 | 18 |
| | Least square means (Standard error) | 0.29(2.637) | 28.22 (37.658) | -0.22(0.20) |

For the change in the concentration of p-Tau in the cerebrospinal fluid at week 52 from the baseline, the concentration of p-Tau consistently tended to be decreased in a dose-dependent manner in the Compound A group compared to the placebo group. There was a statistically significant difference between the Compound A high dose group and the placebo group.

### Preparation Example 1

0.9726 g of magnesium stearate (magnesium stearate, Merck) was added to 174.03 g of maleate of Compound A and the mixture was mixed for 30 minutes. The mixed powder was compression-molded by a roller compactor (TF-LABO (roll pressure 3 MPa), Freund Corporation), and the solid obtained by molding was granulated. 49.51 g of lactose (FlowLac 90, Meggle Japan), 16.50 g of crystalline cellulose (CEOLUS PH302, Asahi Kasei Chemicals) and 6.67 g of croscarmellose sodium (Primellose, DMV Japan) were each sieved through a sieve with an opening of 850 µm and added to 60.0 g of the resulting granulated powder, and the mixture was mixed for 10 minutes. 0.6667 g of magnesium stearate was added to the mixed powder and the mixture was mixed for 30 minutes. The mixed powder was tableted by a tableting machine (HT-P18A, Hata Tekkosho) at a tableting pressure of about 12 kN using a pestle having a double rounded surface with a tablet diameter of 8.5 mm to obtain round uncoated tablets each weighing 250 mg. The uncoated tablets were coated with 8 mg of a coating agent per tablet using a film coater DRC-200 (Powrex), and then a small amount of carnauba wax (Polishing Wax-105, Nippon Wax) was added thereto to give film-coated tablets.

### Preparation Example 2

60.90 g of mannitol (Parteck M200, Merck) and 3.60 g croscarmellose sodium were added to 53.70 g of maleate of Compound A and the mixture was mixed for 10 minutes. 1.80 g of magnesium stearate was added to the mixed powder and the mixture was mixed for 30 minutes. The mixed powder was tableted at a tableting pressure of about 10 kN using a pestle having a double rounded surface with a tablet diameter of 8.5 mm to obtain round uncoated tablets each weighing 250 mg. The uncoated tablets were coated with 8 mg of a coating agent (Opadry 03F44057, 00F440000 (hypromellose 2910: 71.5%, Macrogol 6000: 14.166%, talc: 7.167%, titanium oxide: 7.067%, iron sesquioxide: 0.1%), Colorcon Japan LLC) per tablet, and then a small amount of carnauba wax was added thereto to give film-coated tablets.

### Preparation Example 3

11.11 g of magnesium stearate was added to 1988.89 g of maleate of Compound A and the mixture was mixed for 30 minutes. The mixed powder was compression-molded by a roller compactor, and the solid obtained by molding was granulated. To 107.13 g of the resulting granulated powder were added 26.21 g of mannitol, 7.50 g of ethyl cellulose (ETHOCEL 100FP Premium, The Dow Chemical Company), 3.75 g of crystalline cellulose (CEOLUS KG-1000, Asahi Kasei Chemicals), 3.75 g of crospovidone (Kollidon CL-SF, BASF) and 0.75 g of croscarmellose sodium, and the mixture was mixed for 30 minutes. 0.90 g of magnesium stearate was added to the mixed powder and the mixture was mixed for 5 minutes. The mixed powder was tableted at a tableting pressure of about 7 kN using a pestle having a double rounded surface with a tablet diameter of 8.5 mm to obtain round uncoated tablets each weighing 315 mg. The uncoated tablets were coated with 9 mg of a coating agent per tablet, and then a small amount of carnauba wax was added thereto to give film-coated tablets.

## Claims

1. An agent for preventing or treating tauopathy, comprising 1-(3-(2-(1-Benzothiophen-5-yl)ethoxy)propyl)azetidin-3-ol or a salt thereof as an active ingredient.

2. The agent for preventing or treating tauopathy according to Claim 1, wherein the agent has an effect of reducing the amount of phosphorylated Tau protein.

3. The agent for preventing or treating tauopathy according to Claim 2, wherein the amount of phosphorylated Tau protein is an amount of phosphorylated Tau protein in CSF.

4. The agent for preventing or treating tauopathy according to any one of Claims 1 to 3, wherein the agent has an effect of reducing the amount of amyloid β protein in the brain.

5. The agent for preventing or treating tauopathy according to any one of Claims 1 to 4, wherein the agent has an effect of increasing the amount of amyloid β protein in cerebrospinal fluid.

6. The agent for preventing or treating tauopathy according to any one of Claims 1 to 5, wherein the agent is orally administered in a dose of 100 mg to 400 mg in terms of 1-(3-(2-(1-Benzothiophen-5-yl)ethoxy)propyl)azetidin-3-ol once a day.

7. The agent for preventing or treating tauopathy according to any one of Claim 1 to 5, wherein the agent is orally administered in a dose of 160 mg or 320 mg in terms of 1-(3-(2-(1-Benzothiophen-5-yl)ethoxy)propyl)azetidin-3-ol once a day.

8. The agent for preventing or treating tauopathy according to any one of Claims 1 to 7, wherein the tauopathy is Alzheimer's disease, Probable Alzheimer's disease, Possible Alzheimer's disease, Preclinical Alzheimer's disease, Prodromal Alzheimer's disease, mild cognitive impairment due to Alzheimer's disease (MCI due to AD) or mild cognitive impairment.

9. The agent for preventing or treating tauopathy according to any one of Claims 1 to 7, wherein the tauopathy is Alzheimer's disease, mild cognitive impairment due to Alzheimer's disease (MCI due to AD) or mild cognitive impairment.

10. The agent for preventing or treating tauopathy according to any one of Claims 1 to 7, wherein the tauopathy is Alzheimer's disease.

11. The agent for preventing or treating tauopathy according to any one of Claims 1 to 7, wherein the tauopathy is a disease excluding Alzheimer's disease.

12. The agent for preventing or treating tauopathy according to Claim 11, wherein the disease excluding Alzheimer's disease is mild cognitive impairment due to Alzheimer's disease (MCI due to AD), mild cognitive impairment, frontotemporal dementia, Pick's disease, progressive supranuclear palsy, corticobasal degeneration or Down syndrome.

13. The agent for preventing or treating tauopathy according to claim 11, wherein the disease excluding Alzheimer's disease is mild cognitive impairment due to Alzheimer's disease (MCI due to AD) or mild cognitive impairment.
